# EUROPEAN PATENT SPECIFICATION

(11) **EP 2 979 610 B1**
(45) Date of publication and mention of the grant of the patent: **19.06.2019**
(21) Application number: 14773916.3
(22) Date of filing: 10.01.2014
(51) Int. Cl.: A61B 1/00, A61B 90/50

(54) **ENDOSCOPE SYSTEM**
ENDOSKOPSYSTEM
SYSTÈME D'ENDOSCOPE

(30) Priority: 28.03.2013 US 201361806208 P
(43) Date of publication of application: 03.02.2016
(73) Proprietor: Olympus Corporation, Tokyo 192-8507 (JP)
(72) Inventor: KIKUCHI, Satoru, Hachioji-shi, Tokyo 192-8507 (JP)
(74) Representative: Schicker, Silvia
(86) International application number: PCT/JP2014/050315
(87) International publication number: WO 2014/156218

(56) References cited:
- EP-A1- 0 495 351
- JP-A- 2001 145 634
- JP-A- 2004 229 930
- JP-A- 2005 137 770
- US-A- 5 049 028
- US-A- 5 657 429
- US-A1- 2005 154 261

## Description

### {Technical Field}

The present invention relates to an endoscope system according to the preamble of claim 1. Such an endoscope system is known from US 5,637,429 A.

### {Background Art} '

In the related art, there are known endoscope systems that are employed in laparoscopic surgery or the like and that are configured so that a surgeon performs treatment while observing, by using an endoscope, the state of an affected area which is treated by using a treatment tool inserted into the body cavity of a patient (for example, see Patent Literature 1). The endoscope system disclosed in Patent Literature 1 is configured such that an endoscope inserted into the body cavity acquires some or all of the observation images by using a CCD (Charge Coupled Device) so that a surgeon can perform observation from outside the body.

### {Citation List}

### {Patent Literature}

{PTL 1} Japanese Unexamined Patent Application, Publication No. Hei 10-118006

### {Summary of Invention}

### {Technical Problem}

However, in laparoscopic surgery or the like, as in the case in which tissue in an affected area is removed and the affected area is sutured at the same time by using a linear-stapler treatment tool or in the case in which tubular tissue in an affected area is removed by using a clipping treatment tool while clamping the tubular tissue, it is necessary to check the treatment state by frequently moving the endoscope back and forth between a position at which a close-up viewing field of the affected area can be obtained and a position at which an overlooking viewing field thereof can be obtained. In this case, with a conventional endoscope system, depending on the skill level of an endoscope operator who operates the endoscope, it has been difficult to position the endoscope at an optimal position when repeatedly moving the endoscope in a reciprocating manner back and forth between a position close to the affected area and a position overlooking the affected area, between a lateral position for obtaining a viewing field at the side of the affected area and a frontal position, or the like.

The present invention has been conceived in light of the above-described circumstances, and an object thereof is to provide an endoscope system with which it is possible to position an endoscope in a highly reproducible manner while reducing cumbersome tasks associated with operation by an operator when repeatedly moving the endoscope in a reciprocating manner.

### {Solution to Problem}

Solutions to the problem are described in claim 1 and the dependent claims. An endoscope system including an endoscope main unit is provided with a long, thin inserted portion that is inserted into a body cavity, a main-unit operating portion that is provided at a proximal end of the inserted portion and that can be held by an operator, and an image-acquisition unit that acquires an image of a viewing field at a distal end of the inserted portion; a holder that supports the endoscope main unit, that has a plurality of holder joint portions whose rotating angles can be changed about respective predetermined rotation shafts, and that can change an attitude and a position of the endoscope main unit by changing the rotating angles of the holder joint portions; a holder-angle recording portion that records holder-angle information regarding the rotating angles of the individual holder joint portions; a control portion that controls changes in the rotating angles of the individual holder joint portions; and a switching portion that switches between a manual mode in which the operator holds the main-unit operating portion and changes the attitude and the position of the endoscope main unit and an automatic restoring mode in which the attitude and the position of the endoscope main unit are changed by the control portion, wherein, in the case in which operating mode is switched to the automatic restoring mode from the manual mode by using the switching portion, the control portion reproduces changes in the rotating angles of the individual holder joint portions in time-series in a reverse order based on the holder-angle information recorded in the holder-angle recording portion in the manual mode, thus returning the attitude and the position of the endoscope main unit to an initial state of the manual mode.

With this aspect, the endoscope main unit is held by the holder, and, when the operator holds and manually operates the main-unit operating portion, the rotating angles of the plurality of holder joint portions of the holder are changed, thus changing the attitude and the position of the endoscope main unit. Therefore, when the operator inserts the inserted portion of the endoscope main unit into the body cavity of a patient by manual operation, and acquires an image of a viewing field in front of the inserted portion by using the image-acquisition unit, it is possible to acquire an image of a desired position in the body cavity.

In this case, in the manual mode, when the operator performs observation by inserting the inserted portion into the desired position in the body cavity while changing the attitude and the position of the endoscope main unit, the holder-angle recording portion records the holder-angle information regarding the time-series changes in the rotating angles of the individual holder joint portions, using, as the initial points, the attitude and the position of the endoscope main unit in the initial state in the manual mode.

Then, when the operating mode is switched to the automatic restoring mode from the manual mode by using the switching portion, the control portion reproduces the changes in the rotating angles of the individual holder joint portions in time-series in reverse order based on the holder-angle information recorded in the holder-angle recording portion in the manual mode. By doing so, the endoscope main unit is moved so as to follow, in the opposite direction, the same path as the path on which the endoscope main unit was moved while being manually operated by the operator in the manual mode, thus returning the attitude and the position of the endoscope main unit to the initial state of the manual mode.

Therefore, after the operator observes a desired position in the body cavity by manually operating the endoscope main unit, the endoscope main unit is easily returned to the original attitude and position regardless of the skill level of the operator, and thus, it is possible to obtain a stable viewing field in a highly reproducible manner. Therefore, it is possible to position the endoscope main unit in a highly reproducible manner by reducing cumbersome tasks associated with the operation by the operator when repeatedly moving the endoscope main unit in a reciprocating manner back and forth between a position close to the affected area and a position overlooking the affected area, and so forth.

In the above-described aspect, the plurality of holder joint portions may include a plurality of bending joint portions that can be rotated about the rotation shafts that intersect a longitudinal direction of the holder; a holder-rotating joint portion that can be rotated about the rotation shaft that extends in the longitudinal direction of the holder; and an endoscope-rotating joint portion that rotates the inserted portion about the rotation shaft that extends along an optical axis of the image-acquisition unit.

By employing such a configuration, by individually changing the rotating angles of the plurality of bending joint portions, it is possible to swing the endoscope main unit in a direction that intersects the optical axis, to move the endoscope main unit forward and backward in the optical-axis direction, or to translationally move the endoscope main unit in a direction parallel to the optical axis or the like. In addition, by changing the rotating angles of the holder-rotating joint portions, it is possible to move the viewing field of the inserted portion in a direction perpendicular to the optical axis. In addition, by changing the rotating angles of the endoscope-rotating joint portions, it is possible to rotate the viewing field of the inserted portion about the optical axis. Therefore, it is possible to freely manipulate the inserted portion of the endoscope main unit in the body cavity.

In the above-described aspect, the endoscope main unit may include a plurality of distal-end joint portions that are provided at a distal-end portion of the inserted portion and whose rotating angles can be changed about respective predetermined rotation shafts that intersect the longitudinal direction; and a distal-end operating portion that changes the rotating angles of the respective distal-end joint portions in response to an operation by the operator, wherein the endoscope system comprises a distal-end-angle recording portion that records distal-end-angle information regarding time-series changes in the rotating angles of the respective distal-end joint portions that are changed by the distal-end operating portion, and wherein, in the case in which the operating mode is switched to the automatic restoring mode from the manual mode by using the switching portion, the control portion reproduces changes in the rotating angles of the respective distal-end joint portions in time-series in a reverse order, in synchronization with reproducing the changes in the rotating angles of the individual holder joint portions in a reverse order, based on the distal-end-angle information recorded in the distal-end-angle recording portion in the manual mode, thus returning the angle of the inserted portion to the initial state of the manual mode.

By employing such a configuration, by bending the distal end of the inserted portion in the direction that intersects the optical axis by using the plurality of distal-end joint portions, it is possible to easily ensure the use of viewing fields on the back side of tissue and in narrow spaces without having to expand the viewing field to cover a large area by moving the entire inserted portion.

In the above-described aspect, the holder joint portion may include rotational drive portions that are rotationally driven about the rotation shafts; and an information outputting portion that outputs the holder-angle information of the rotational drive portions, wherein the control portion stops control of the rotational drive in the manual mode, and stops outputting of the holder-angle information from the information outputting portion in the automatic restoring mode.

By employing such a configuration, in the manual mode, the operator can easily manually rotate the rotating portion about the rotation shaft without being affected by the driving portion. In addition, in the automatic restoring mode, it is possible to reduce power consumption by an amount equivalent to the reduction in power achieved by stopping the output of the holder-angle information from the information outputting portion.

In the above-described aspect, the switching portion may stop the control of the individual holder joint portions by the control portion, may also stop recording of the holder-angle information by the holder-angle recording portion, and may switch the operating mode among a non-recording mode in which the operator holds the main-unit operating portion and changes the attitude and the position of the endoscope main unit, the manual mode, and the automatic restoring mode.

By employing such a configuration, the operator can manually operate the endoscope main unit without being affected by the control portion while suppressing the power required to change the rotating angles of the holder joint portions and also keeping the memory capacity of the holder-angle recording portion low.

The above-described aspect may be provided with an image recording portion that records an image of a viewing field at the distal end, acquired by the image-acquisition unit; and an image comparing portion that compares the image recorded by the image recording portion in the manual mode and an image of the viewing field at the distal end, acquired by the image-acquisition unit in the automatic restoring mode, wherein, in the case in which the image comparing portion judges that a difference between a characteristic of the image of the viewing field at the distal end, acquired in the automatic restoring mode, and a characteristic of the image recorded by the image recording portion in the manual mode is equal to or greater than a predetermined threshold, the control portion stops reproducing the changes in the rotating angles of the holder joint portions in the reverse order.

By employing such a configuration, it is possible to prevent the inserted portion from coming into contact with a treatment tool or the like in the case in which there have been changes in the environment in the body cavity, states of other treatment tools, or the like between the manual mode and the automatic restoring mode.

In the above-described aspect, a plurality of the endoscope main units in which types, numbers, and/or positions of the holder joint portions are different may have unit-specific information in accordance with the types, numbers, and/or positions of the holder joint portions, the holder may be provided with an identifying portion that identifies the unit-specific information of the supported endoscope apparatus, and the control portion may control changes in the rotating angles of the individual holder joint portions based on the unit-specific information identified by the identifying portion.

By employing such a configuration, when an endoscope main unit is supported by the holder, the identifying portion identifies the unit-specific information of that endoscope main unit, and the control portion controls changes in the rotating angles of the individual holder joint portions based on that unit-specific information. Therefore, in the automatic restoring mode, it is possible to perform operation in accordance with the types, numbers, and/or positions of the holder joint portions of each endoscope main unit.

With this aspect, in the recording step, when the endoscope main unit supported by the holder is held by the operator and manually operated, the holder-angle information regarding the time-series changes in the rotating angles of the individual holder joint portions is recorded, using, as
the initial points, the attitude and the position of the endoscope main unit in the initial state.

Subsequently, in the automatic restoring step, by reproducing the changes in the rotating angles of the individual holder joint portions in time-series in reverse order based on the holder-angle information recorded in the recording step, the endoscope main unit is moved so as to follow, in the opposite direction, the path on which the endoscope main unit was moved while being held and manually operated by the operator, thus returning the attitude and the position of the endoscope main unit to the initial state of the recording step.

Therefore, after the operator observes a desired position in the body cavity by manually operating the endoscope main unit, the endoscope main unit is easily returned to the original attitude and position regardless of the skill level of the operator, and thus, it is possible to obtain a stable viewing field in a highly reproducible manner. Therefore, it is possible to position the endoscope in a highly reproducible manner by reducing cumbersome tasks associated with the operation by the operator when repeatedly moving the endoscope main unit in a reciprocating manner back and forth between a position close to the affected area and a position overlooking the affected area, and so forth.

### {Advantageous Effects of Invention}

With the present invention, an advantage is afforded in that it is possible to position an endoscope in a highly reproducible manner while reducing cumbersome tasks associated with operation by an operator when repeatedly moving the endoscope in a reciprocating manner.

### {Brief Description of Drawings}

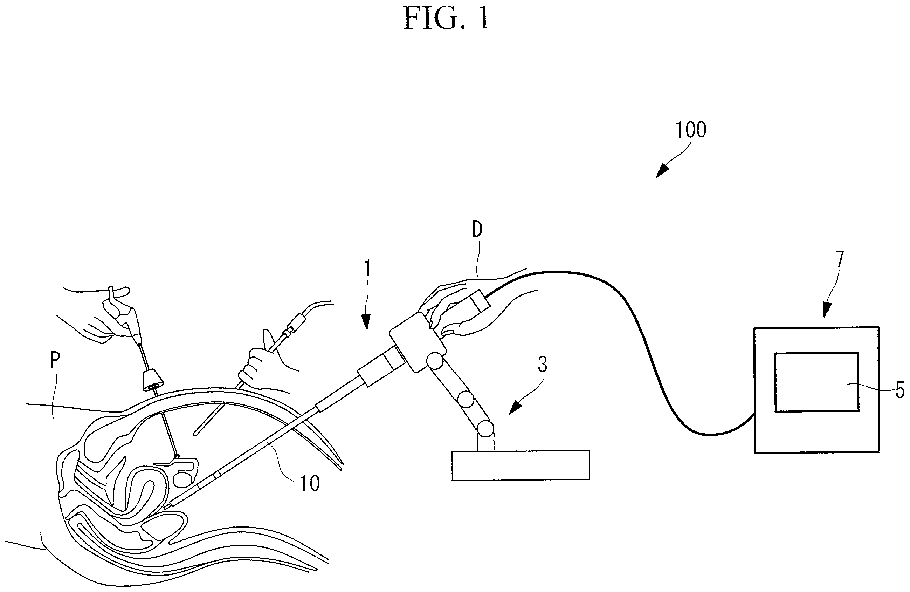

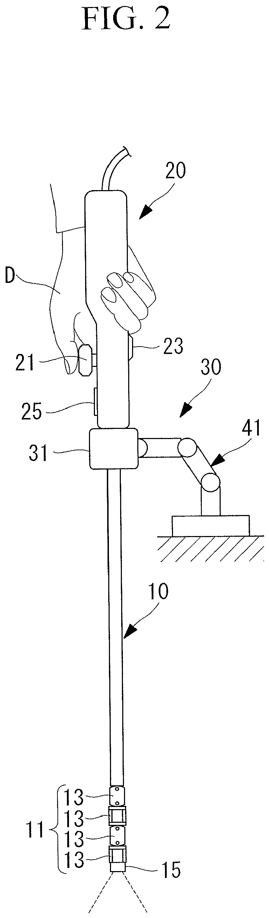

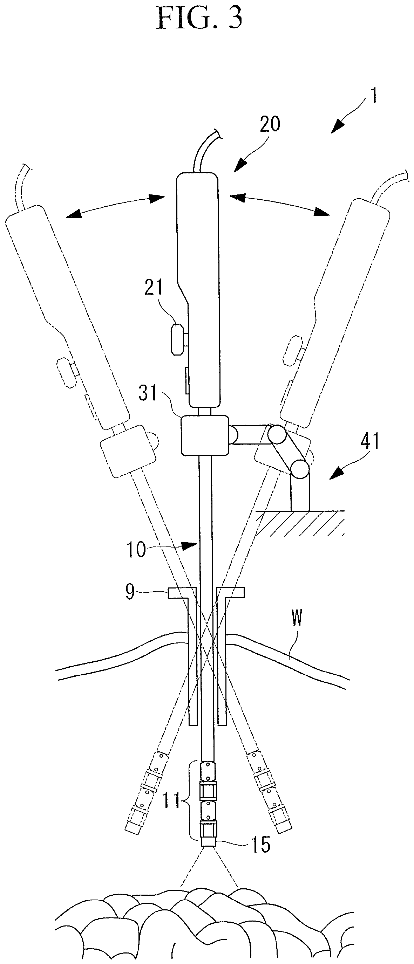

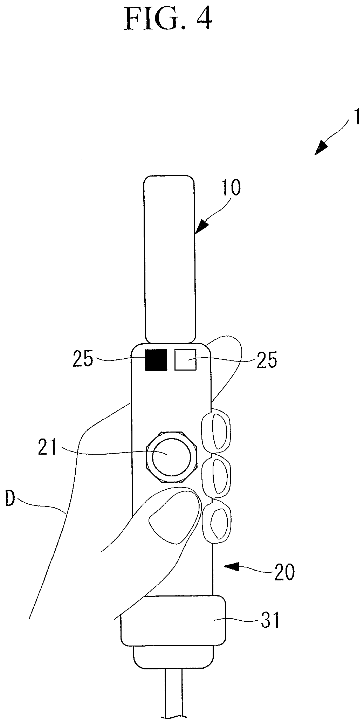

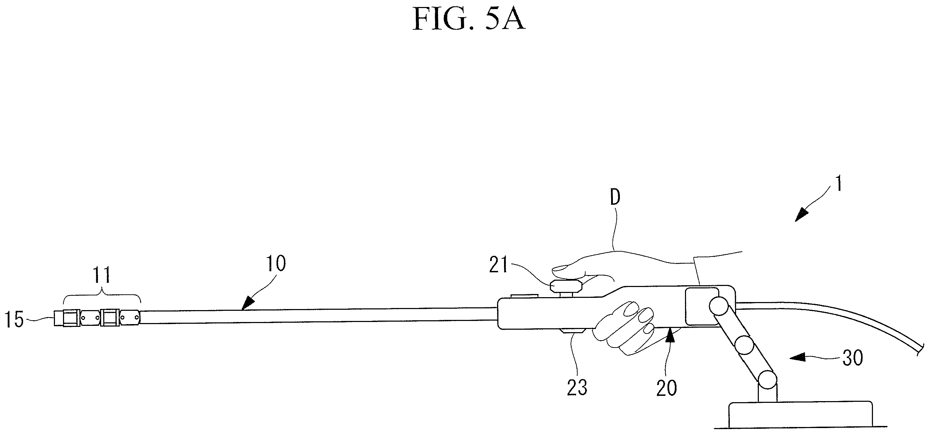

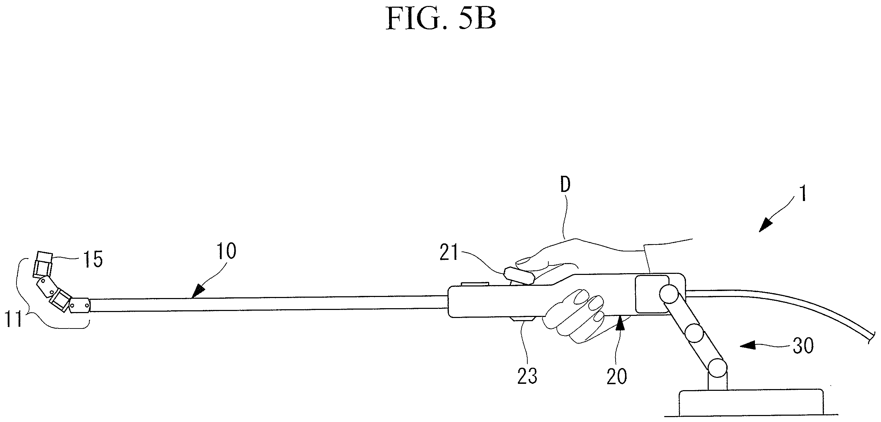

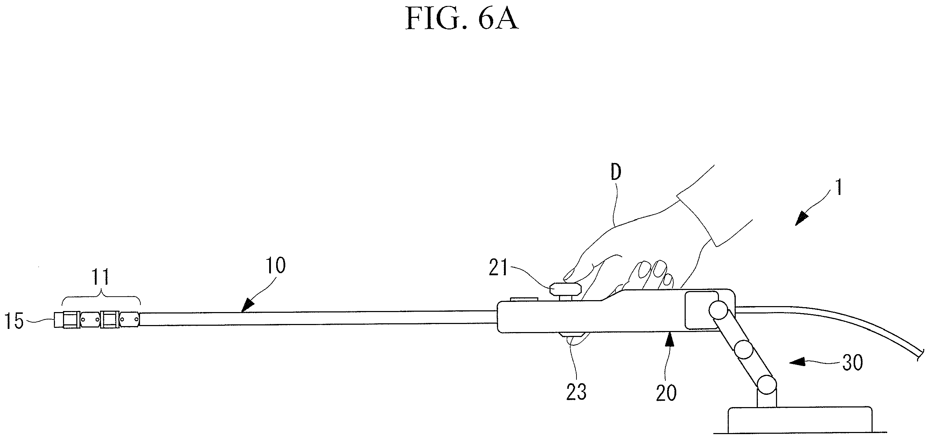

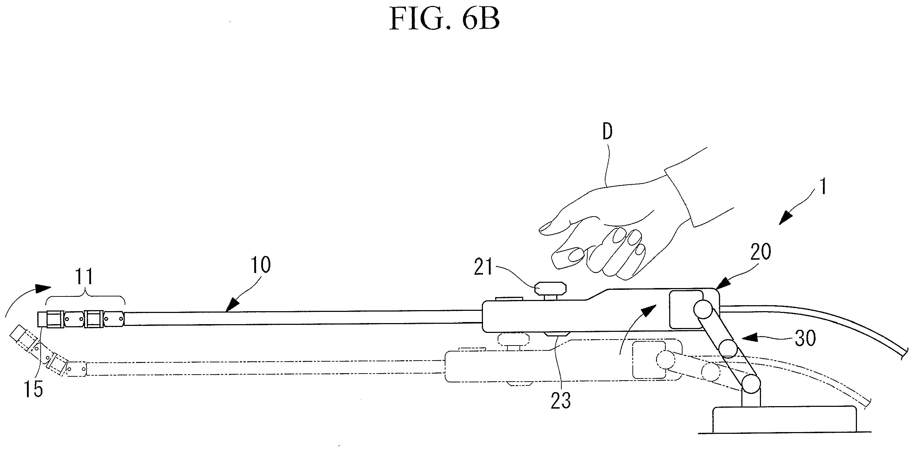

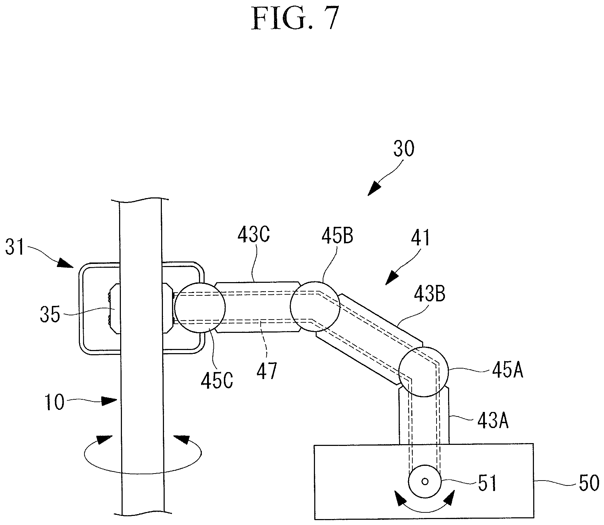

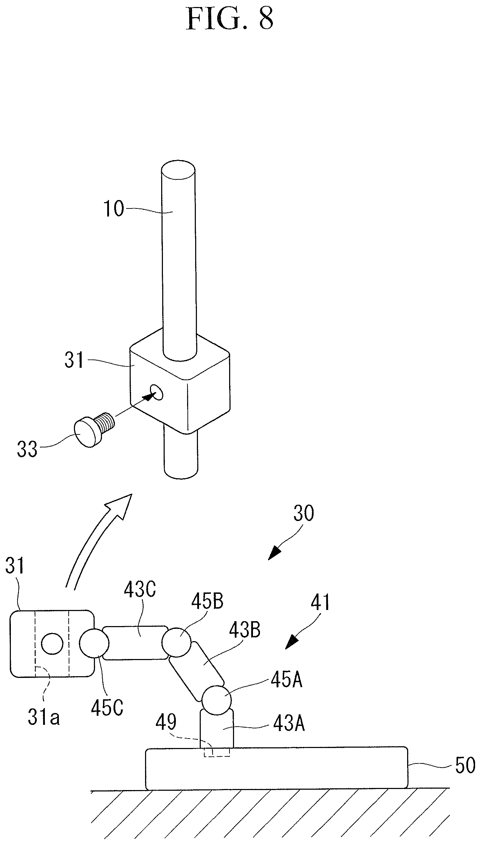

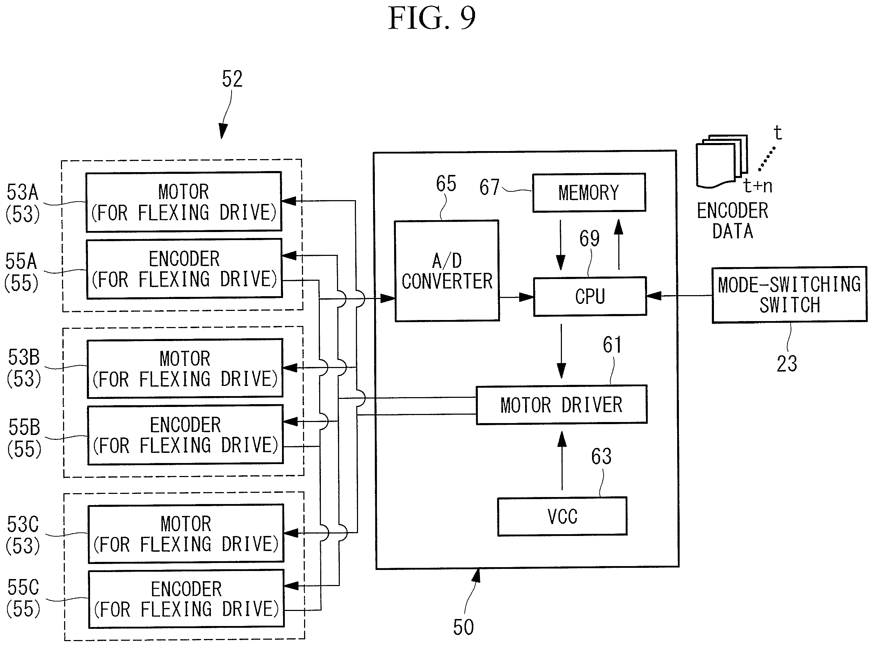

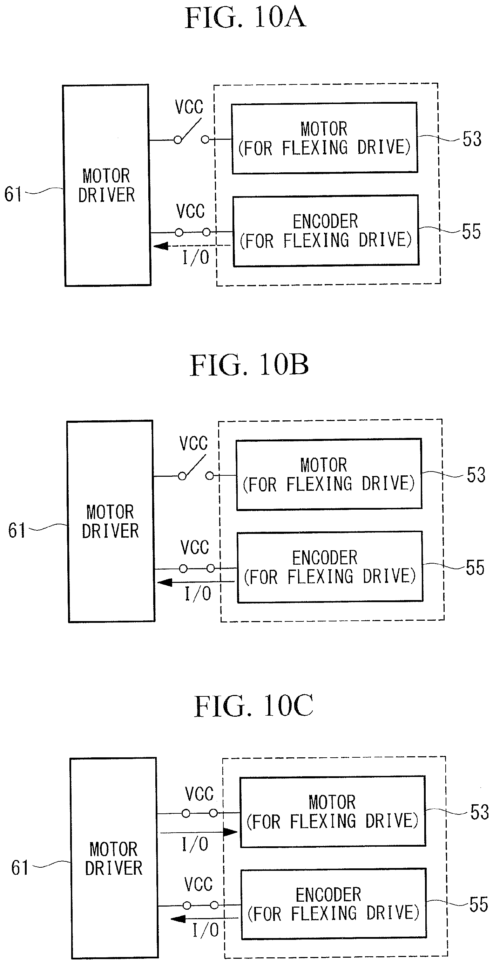

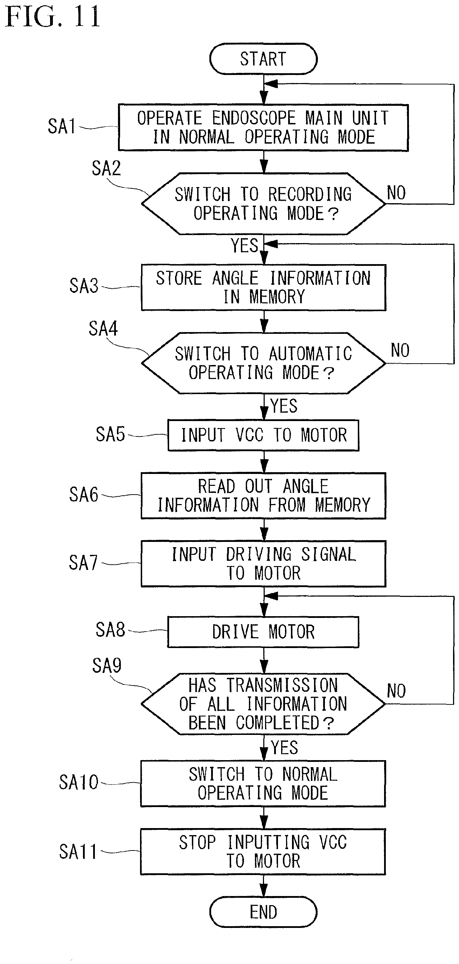

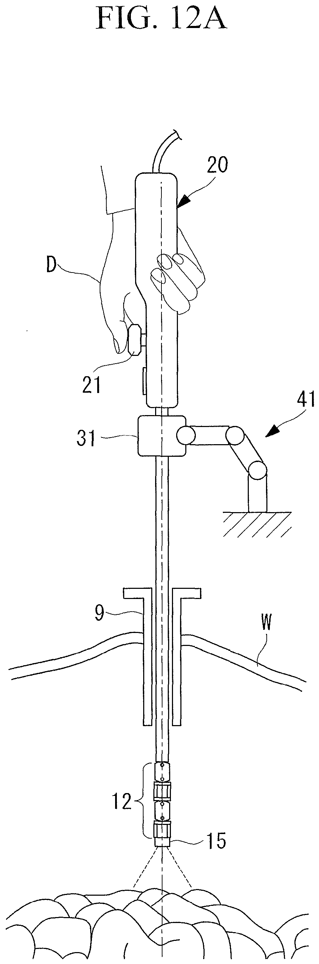

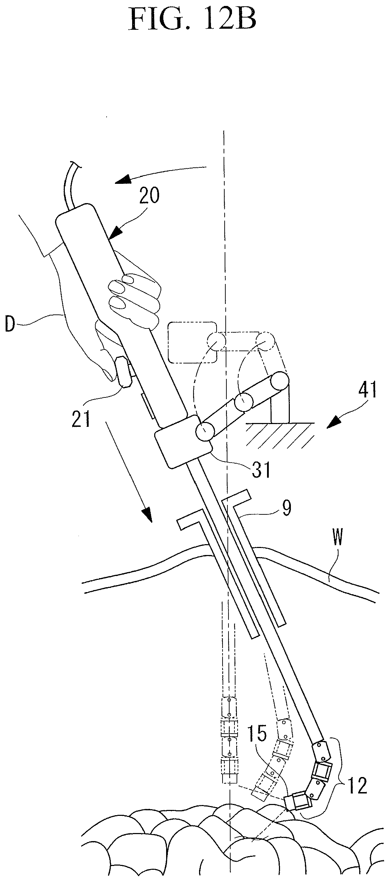

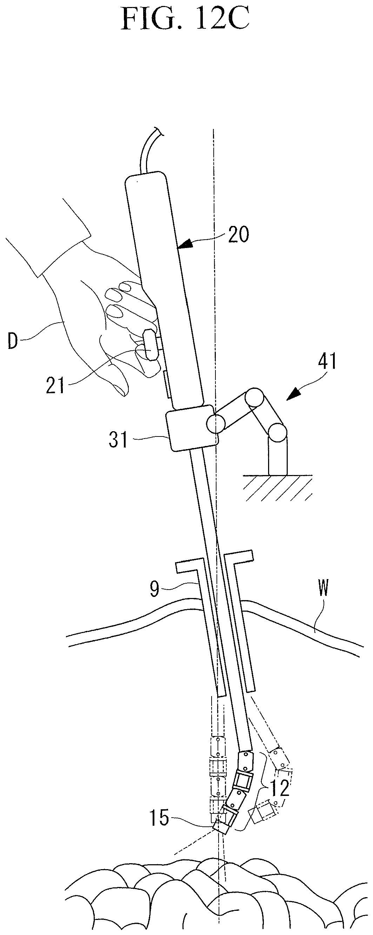

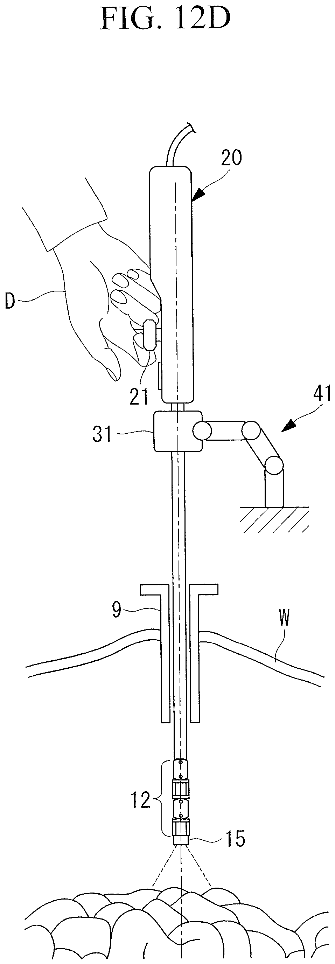

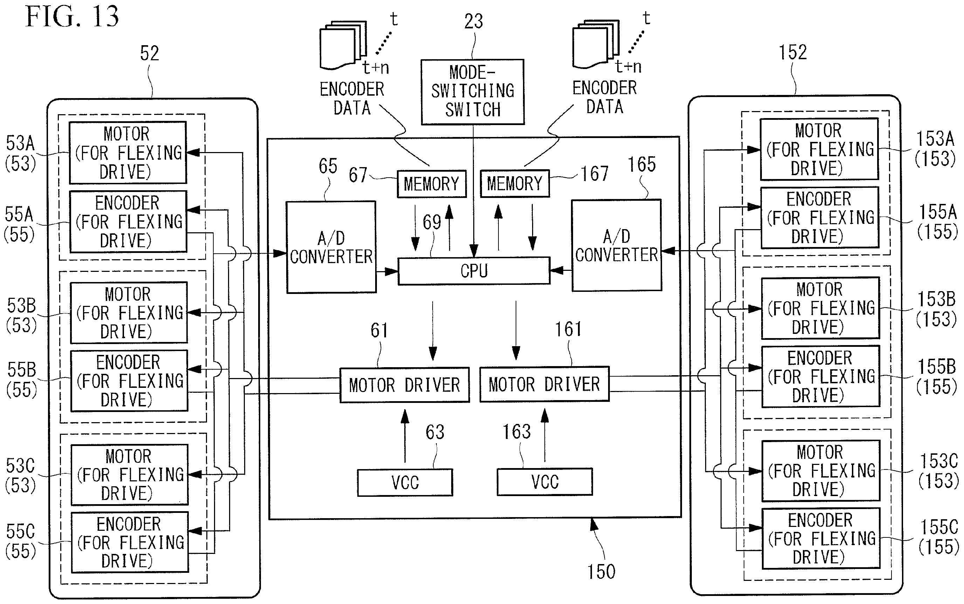

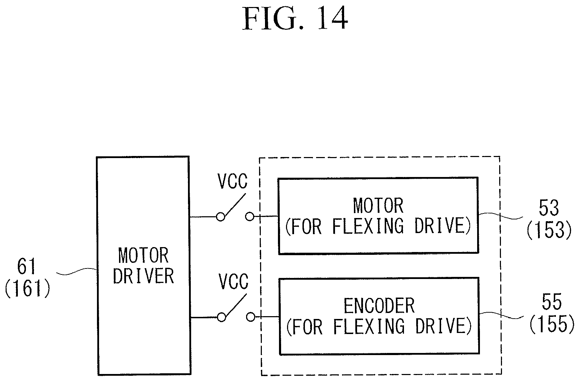

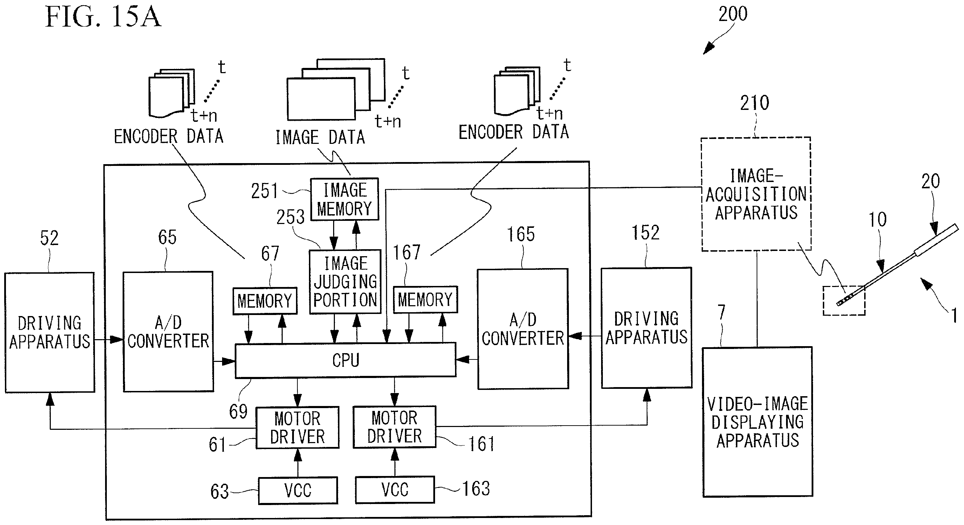

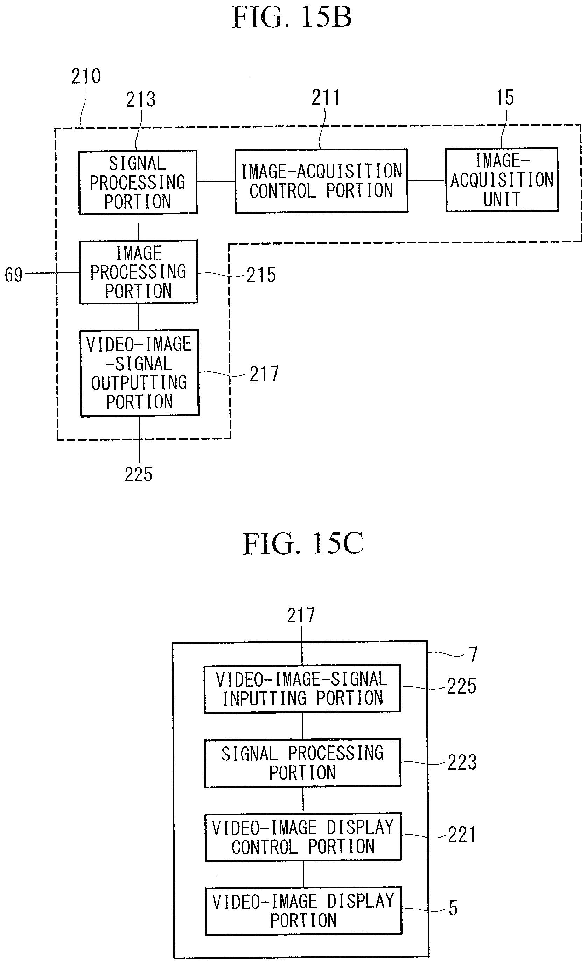

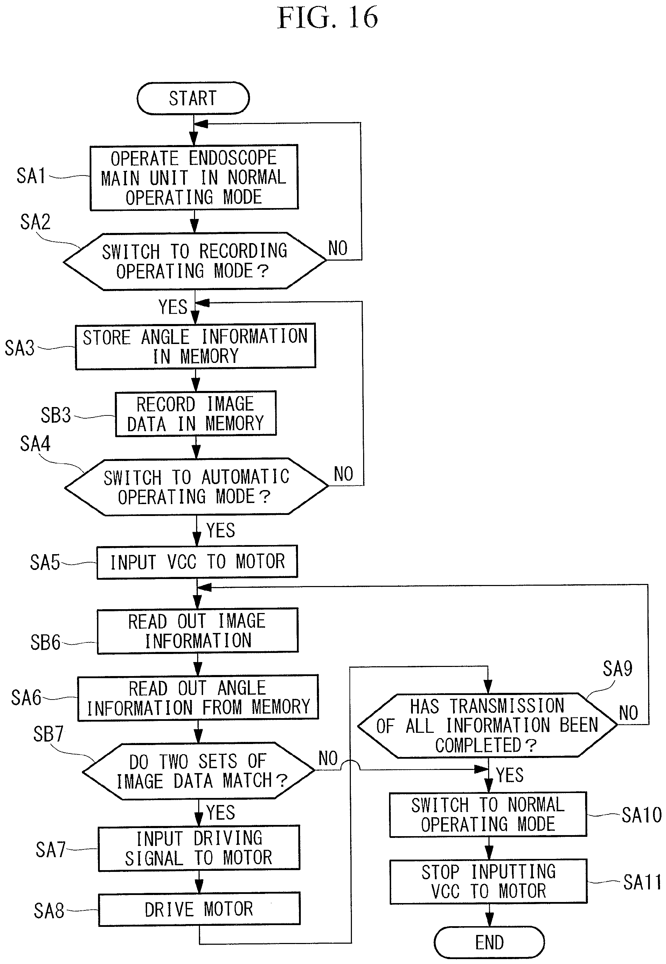

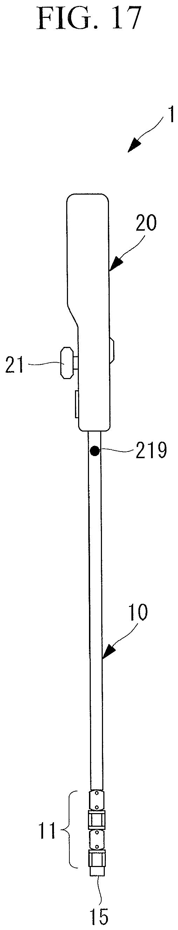

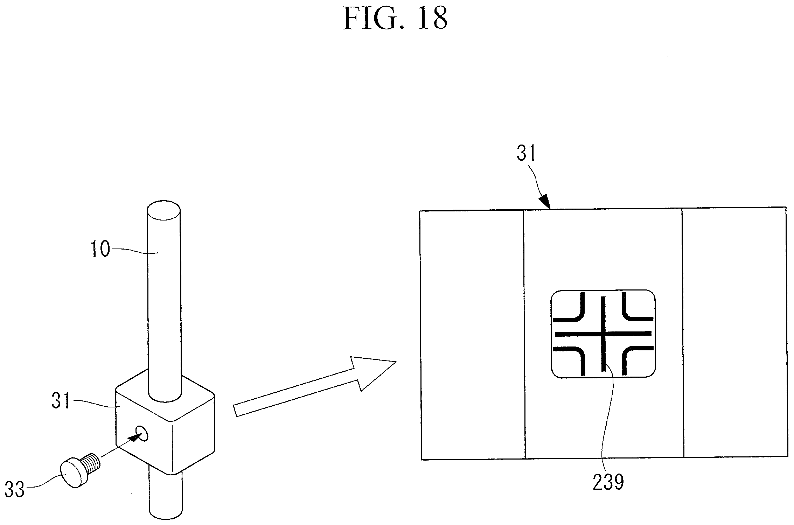

{Fig. 1} Fig. 1 is an overall configuration diagram showing an endoscope system according to a first embodiment of the present invention.
{Fig. 2} Fig. 2 is an enlarged view of the endoscope system in Fig. 1.
{Fig. 3} Fig. 3 is a diagram showing the process of operating an endoscope main unit in Fig. 2 in a body cavity.
{Fig. 4} Fig. 4 is an enlarged view of an operating portion of the endoscope main unit in Fig. 2.
{Fig. 5A} Fig. 5A is a diagram showing a state in which a shaft of the endoscope main unit in Fig. 2 is extended in a straight line.
{Fig. 5B} Fig. 5B is a diagram showing a state in which the distal-end portion of the shaft of the endoscope main unit in Fig. 5A is bent.
{Fig. 6A} Fig. 6A is a diagram showing a state in which a mode-switching switch of the endoscope main unit in Fig. 2 is continuously pressed.
{Fig. 6B} Fig. 6B is a diagram showing a state in which a finger is released from the mode-switching switch of the endoscope main unit in Fig. 2.
{Fig. 7} Fig. 7 is a longitudinal sectional view of a holding portion and a supporting portion of an arm.
{Fig. 8} Fig. 8 is a diagram for explaining a configuration for securing a shaft in a position-aligned state by using the holding portion in Fig. 7.
{Fig. 9} Fig. 9 is a block diagram for explaining the configuration of a control device.
{Fig. 10A} Fig. 10A is a diagram showing the relationship between a motor driver and a motor and that between the motor driver and an encoder in a normal operating mode.
{Fig. 10B} Fig. 10B is a diagram showing the relationship between the motor driver and the motor and that between the motor driver and the encoder in a recording operating mode.
{Fig. 10C} Fig. 10C is a diagram showing the relationship between the motor driver and the motor and that between the motor driver and the encoder in an automatic operating mode.
{Fig. 11} Fig. 11 is a flowchart for explaining an operating method for the endoscope system in Fig. 1.
{Fig. 12A} Fig. 12A is a diagram showing a state in which the
   shaft is inserted into the body cavity in the normal operating mode.
{Fig. 12B} Fig. 12B is a diagram showing a state in which the shaft is being manipulated in the body cavity in the recording operating mode.
{Fig. 12C} Fig. 12C is a diagram showing a state in which the attitude and the position of the endoscope main unit are automatically restored in the automatic operating mode.
{Fig. 12D} Fig. 12D is a diagram showing a state in which the automatic restoration of the endoscope main unit has been completed.
{Fig. 13} Fig. 13 is a block diagram for explaining the configuration of a control device of an endoscope system according to a second embodiment of the present invention.
{Fig. 14} Fig. 14 is a diagram showing the relationship between a motor driver and a motor and that between the motor driver and an encoder, in a manual operating mode, included in an endoscope system according to a modification of the first embodiment and the second embodiment of the present invention.
{Fig. 15A} Fig. 15A is a block diagram showing an endoscope system according to a third embodiment of the present invention.
{Fig. 15B} Fig. 15B is a block diagram for explaining the configuration of an image-acquisition apparatus in Fig. 15A.
{Fig. 15C} Fig. 15C is a block diagram for explaining the configuration of a video-image displaying apparatus in Fig. 15A.
{Fig. 16} Fig. 16 is a flowchart for explaining an operating method for the endoscope system in Fig. 15A.
{Fig. 17} Fig. 17 is a diagram showing an ID recording portion provided in a shaft of an endoscope system according to modifications of the individual embodiments of the present invention.
{Fig. 18} Fig. 18 is a diagram showing an ID reading portion provided in an arm of the endoscope system according to the modifications of the individual embodiments of the present invention.

### {Description of Embodiments}

### {First Embodiment}

An endoscope system according to a first embodiment of the present invention will be described below with reference to the drawings.

As shown in Fig. 1, an endoscope system 100 according to this embodiment is provided with an endoscope main unit 1 that acquires an image of the interior of the body cavity of a patient P, an endoscope-holding apparatus 3 that holds the endoscope main unit 1 and that assists in the operation of the endoscope main unit 1 by an operator D, and a video-image displaying apparatus 7 that has a monitor 5 that displays the image acquired by the endoscope main unit 1.

As shown in Fig. 2, the endoscope main unit 1 is provided with a long, thin substantially cylindrical shaft (inserted portion) 10 that is inserted into the body cavity of the patient P, an operating portion (main-unit operating portion) 20 that is provided at the proximal end of the shaft 10 and that is held by the operator D, and an image-acquisition apparatus (not shown) that has an image-acquisition unit 15 that acquires an image of the viewing field at the distal end of the shaft 10.

As shown in Fig. 3, the shaft 10 is configured so that it can be inserted into the body via a trocar 9 piercing a body wall W, such as an abdominal wall or the like, of the patient P. In addition, the shaft 10 is configured so as to be supported by the trocar 9 at an intermediate position in the longitudinal direction such that the distal-end portion thereof can be moved by using the trocar 9 as a fulcrum.

In addition, the shaft 10 includes, at the distal-end portion, a bending portion 11 that has, for example, a segmented structure and that is bendable in a direction that intersects the longitudinal direction of the shaft 10. The bending portion 11 is formed of a plurality of (four in this embodiment) distal-end joint portions 13 whose rotating angles can be changed about predetermined respective rotation shafts that are perpendicular to the longitudinal direction.

The distal-end joint portions 13 are configured so that their rotating angles can be changed about the respective rotation shafts by means of rotational driving with motors (not shown). By doing so, distal-end surface of the shaft 10 can be made to face a direction that intersects the longitudinal direction.

An image-acquisition unit 15 of the image-acquisition apparatus is built into the distal end of the shaft 10. The image-acquisition unit 15 has a viewing field that faces forward at the distal-end surface of the shaft 10 and is configured so that the orientation of the viewing field can be changed in accordance with the orientation of the distal-end surface of the shaft 10 by bending the bending portion 11.

The operating portion 20 is formed in a substantially cylindrical shape that is larger than the shaft 10, and the operator D can hold and operate it with one hand in a state in which the shaft 10 side is made to face forward. The operating portion 20 is provided with a joystick (distal-end operating portion) 21 that changes the rotating angles of the individual distal-end joint portions 13 of the shaft 10, a mode-switching switch (switching portion) 23 that switches the operating mode of the endoscope main unit 1, and a state-displaying indicator 25 that displays the set state of the operating modes.

As shown in Fig. 4, the joystick 21 is disposed closer to the shaft 10 in the operating portion 20, and is configured so that the operator D can operate the operating portion 20 with his/her thumb while holding the operating portion 20. The joystick 21 is configured so that the rotating angles of the individual distal-end joint portions 13 can be changed in accordance with the tilting angle of the joystick 21.

In addition, the joystick 21 is configured so as to allow the bending portion 11 of the shaft 10 to be bent in the direction in which the operator D tilts the joystick 21. For example, as shown in Figs. 5A and 5B, when the joystick 21 is tilted toward the proximal end of the operating portion 20 in a state in which the operator D is holding the operating portion 20 so that the shaft 10 side faces forward, the distal-end portion of the shaft 10 can be bent upward. In addition, the distal-end portion of the shaft 10 can be bent downward when the joystick 21 is tilted toward the distal end of the operating portion 20, and the distal-end portion of the shaft 10 can be bent left- or rightward when the joystick 21 is tilted in a width direction (left- or rightward) of the operating portion 20.

As shown in Figs. 5A and 5B, the mode-switching switch 23 is disposed at a surface of the operating portion 20 on the opposite side of the joystick 21, and is configured so that the operator D can press the mode-switching switch 23 with his/her index finger while holding the operating portion 20. The mode-switching switch 23 is configured to allow switching among, for example, three modes, namely, a normal operating mode, a recording operating mode (manual mode), and an automatic operating mode (automatic restoring mode).

For example, when the operator D presses the mode-switching switch 23 in the normal operating mode, the operating mode is switched to the recording operating mode while the mode-switching switch 23 is kept in the pressed state, and the operating mode is switched to the automatic operating mode when the operator D releases his/her finger. Also, the mode-switching switch 23 is configured so as to switch the operating mode to the normal operating mode when the automatic operating mode is completed. Fig. 6A shows a state in which the operating mode is switched to the recording operating mode when the operator D keeps pressing the mode-switching switch 23, and Fig. 6B shows a state in which the operating mode is switched to the automatic operating mode when the operator D releases his/her finger from the mode-switching switch 23.

The state-displaying indicator 25 is disposed at the distal end of the operating portion 20 at the same surface as the joystick 21. The state-displaying indicator 25 is configured so as to take different lighting forms for the normal operating mode, the recording operating mode, and the automatic operating mode, respectively. For example, both lights on the left and right sides are turned on in the normal operating mode, the light on the left side facing the shaft 10 is turned on and the light on the right side is turned off in the recording operating mode, and the light on the right side facing the shaft 10 is turned on and the light on the left side is turned off in the automatic operating mode.

As shown in Fig. 7, the endoscope-holding apparatus 3 is provided with an arm (holder) 30 that supports the endoscope main unit 1 and a control device 50 that controls the arm 30. The arm 30 is provided with a holding portion 31 that holds the endoscope main unit 1 and a supporting portion 41 that supports the holding portion 31 and that can be bent in the direction perpendicular to the longitudinal direction.

The holding portion 31 and the supporting portion 41 are provided with a plurality of arm-joint portions (holder joint portions) 35, 45A, 45B, 45C, and so forth whose rotating angles can be changed about predetermined respective rotation shafts, and, by changing the rotating angles of the arm-joint portions 35, 45A, 45B, 45C, and so forth, it is possible to change the attitude and the position of the endoscope main unit 1.

The holding portion 31 can hold, for example, the proximal end portion of the shaft 10 or the operating portion 20. As shown in Fig. 8, the holding portion 31 has a through-hole 31a into which the shaft 10 of the endoscope main unit 1 can be fitted, and is configured so that the shaft 10 fitted into the through-hole 31a can be secured by means of a screw 33 in a state in which positions thereof in the longitudinal direction and the circumferential direction are set. As shown in Fig. 7, the holding portion 31 is provided with an endoscope-rotating joint portion (holder joint portion) 35 that makes the shaft 10 fitted into the through-hole 31a rotate about a rotation shaft aligned with the optical-axis direction of the image-acquisition unit 15.

The supporting portion 41 is formed of a plurality of (three in this embodiment) frames 43A, 43B, and 43C and a plurality of (three in this embodiment) bending joint portions (holder joint portions) 45A, 45B, and 45C whose rotating angles can be changed about rotation shafts that are perpendicular to the longitudinal direction of the arm 30 and that are alternately coupled in order from the proximal end toward the distal-end side of the supporting portion 41. The bending joint portion 45C, which is placed at the most distal end, is connected to the holding portion 31, and the frame 43A, which is placed at the rear most end, is connected to the control device 50.

In addition, the supporting portion 41 is provided with a plurality of wires 47, one end of which is connected to the endoscope-rotating joint portion 35 of the holding portion 31 and the other end of which is connected to, via the bending joint portions 45A, 45B, and 45C, a pulley 51 that is accommodated in the control device 50. When the tensions of the wires 47 are changed by rotation of the pulley 51, it is possible to bend the supporting portion 41 in the direction that intersects the longitudinal direction by changing the rotating angles of the bending joint portions 45A, 45B, and 45C, or it is possible to rotate the shaft 10 about the rotation shaft by using the endoscope-rotating joint portion 35.

In addition, the supporting portion 41 is provided with an arm-rotating joint portion (holder-rotating joint portion) 49 that can be rotated about a rotation shaft that extends in the direction parallel to the longitudinal direction of the arm 30, and the frame 43A disposed at the rear most end is secured to the control device 50 via the arm-rotating joint portion 49. The supporting portion 41 is configured so that the arm 30 can be rotated in the circumferential direction by the arm-rotating joint portion 49 centered on the rotation shaft thereof. In the following, the endoscope-rotating joint portion 35, the bending joint portions 45A, 45B, and 45C, and the arm-rotating joint portion 49 are referred to as arm-joint portions when described collectively.

As shown in Fig. 9, the pulley 51, motors (rotational drive portions) 53 that rotate the arm-joint portions 35, 45A, 45B, 45C, and 49 about the respective rotation shafts, and encoders (information outputting portions) 55 that convert the rotating angles of the individual motors 53 to electrical signals and that output them in the form of arm-angle information (holder-angle information) are accommodated in an arm-driving apparatus 52 individually for the arm-joint portions 35, 45A, 45B, 45C, and 49. Fig. 9 shows, as
examples, motors 53A, 53B, and 53C and encoders 55A, 55B, and 55C (all of which are for flexing drive) of the bending joint portions 45A, 45B, and 45C disposed in the arm 30.

The control device 50 is provided with a motor driver 61 that drives the individual motors 53 and the individual encoders 55, a VCC supplying portion 63 that outputs VCC (power-source voltage) to be supplied to the motors 53 and the encoders 55 from the motor driver 61, an A/D converter 65 that converts the arm-angle information output from the encoders 55 from analog signals and that outputs them in the form of digital signals, a memory (holder-angle recording portion) 67 that records arm-angle information (encoder data)for each of the arm-joint portions 35, 45A, 45B, 45C, and 49, and a CPU (Central Processing Unit, control portion) 69 that controls changes in the rotating angles of each of the arm-joint portions 35, 45A, 45B, 45C, and 49 via the motor driver 61, the memory 67, and so forth.

In the normal operating mode, the CPU 69 stops supplying VCC to be applied to the motors 53 from the motor driver 61, as shown in Fig. 10A, while, on the other hand, allowing the VCC to be supplied to the encoders 55 from the motor driver 61; however, the inputs of the arm-angle information from the encoders 55 are stopped (Input/Output is kept open). In addition, in the recording operating mode, the CPU 69 stops supplying VCC to the motors 53 from the motor driver 61, while, on the other hand, allowing VCC to be supplied to the encoders 55 from the motor driver 61, receives the inputs of the arm-angle information from the encoders 55, and transmits the information to the memory 67.

In addition, in the automatic operating mode, the CPU 69 allows VCC to be supplied to the motors 53 and the encoders 55 from the motor driver 61. In addition, in the automatic operating mode, the CPU 69 reads out, in time-series, the arm-angle information recorded in the memory 67 in the recording operating mode and transmits the information to the motors 53 from the motor driver 61, and also receives the inputs of the arm-angle information from the encoders 55 and transmits the information to the memory 67.

Specifically, when the recording operating mode is switched to the automatic operating mode via the mode-switching switch 23, the CPU 69 makes the motor driver 61 and the motors 53 reproduce the changes in the rotating angles of each of the arm-joint portions 35, 45A, 45B, 45C, and 49, which have been recorded in the memory 67 in the recording operating mode, in time-series in reverse order. By doing so, the CPU 69 makes the attitude and the position of the endoscope main unit 1 return to the initial state of the recording operating mode.

Next, as shown in the flowchart in Fig. 11, the operating method for the endoscope system according to this embodiment includes a recording step SA3 of recording the arm-angle information regarding the rotating angles of each of the arm-joint portions 35, 45A, 45B, 45C, and 49 when the attitude and the position of the endoscope main unit 1 is changed by the operator D holding the endoscope main unit 1 and an automatic restoring step SA8 of making the attitude and the position of the endoscope main unit 1 return to the initial state of the recording step SA3 by reproducing the changes in the rotating angles of each of the arm-joint portions 35, 45A, 45B, 45C, and 49 in time-series in the reverse order based on the arm-angle information recorded in the recording step SA3.

The operation of the thus-configured endoscope system 100 and the effects of the operating method thereof will be described with reference to the flowchart in Fig. 11. The endoscope system 100 according to this embodiment is operated by the operator D holding the endoscope main unit 1 supported by the arm 30. When the operator D moves the endoscope main unit 1, rotating angles of the endoscope-rotating joint portion 35 of the holding portion 31, the bending joint portions 45A, 45B, and 45C of the supporting portion 41 and the arm-rotating joint portion 49 about the respective rotation shafts are changed, which makes it possible to change the attitude and the position of the endoscope main unit 1 assisted by the arm 30.

In order to observe a desired position in the body cavity by using the endoscope system 100 and the operating method thereof, first, the operator D holds and operates the endoscope main unit 1, which is supported by the arm 30, in the normal operating mode (Step SA1), and inserts the shaft 10 into the body via the trocar 9 piercing the body wall W, such as an abdominal wall or the like, of the patient P, as shown in Fig. 12A. Then, an image of a viewing field in front of the distal end of the shaft 10 is acquired by using the image-acquisition unit 15.

Subsequently, the operator D presses the mode-switching switch 23 to switch the operating mode to the recording operating mode ("YES" in Step SA2), and operates the endoscope main unit 1 while keeping the mode-switching switch 23 pressed. For example, as shown in Fig. 12B, the operator D operates the endoscope main unit 1 so that an image of a desired viewing field can be obtained by inserting the shaft 10 of the endoscope main unit 1 to a deeper position in the body cavity and by moving the distal-end portion of the shaft 10 in the direction that intersects the longitudinal direction by using the trocar 9 as a fulcrum.

In the endoscope-holding apparatus 3, by switching to the recording operating mode, the CPU 69 allows VCC to be supplied to the encoders 55 from the motor driver 61, and the arm-angle information of the respective arm-joint portions 35, 45A, 45B, 45C, and 49 output from the encoders 55 is recorded in the memory 67 (Step SA3, recording step). By doing so, the arm-angle information associated with changes in the rotating angles of the individual arm-joint portions 35, 45A, 45B, 45C, and 49 is recorded in the memory 67 at certain time intervals, using, as the initial points, the arm-angle information of the individual arm-joint portions 35, 45A, 45B, 45C, and 49 at the time when the recording operating mode is started.

At this time, as shown in Fig. 12B, the rotating angles of the individual distal-end joint portions 13 of the shaft 10 are changed when the operator D operates the joystick 21, and thus, the bending portion 11 is bent in a direction and at an angle in accordance with the titling direction and the tilting angle of the joystick 21. By doing so, it is possible to easily ensure the use of viewing fields located at the back side of tissue and in narrow spaces by directing the viewing field of the image-acquisition unit 15 into a desired direction without having to move the entire shaft 10.

Subsequently, when image acquisition at a desired observation position in the body cavity is completed, the operator D switches the operating mode to the automatic operating mode by releasing his/her finger from the mode-switching switch 23 (Step SA4), and then releases his/her hand from the operating portion 20. In the endoscope-holding apparatus 3, the CPU 69 allows VCC to be supplied to the motors 53 and the encoders 55 from the motor driver 61 (Step SA5) .

In addition, the CPU 69 reads out, in time-series, the arm-angle information recorded in the memory 67 in the recording operating mode (Step SA6). Then, the read-out arm-angle information is transmitted, in time-series, to the motors 53 from the motor driver 61 (Step SA7), and the individual motors 53 are driven based on the arm-angle information (Step SA8, automatic restoring step).

By doing so, as shown in Fig. 12C, the rotating angles of each of the arm-joint portions 35, 45A, 45B, 45C, and 49 in the recording operating mode are reproduced in time-series in reverse order, and thus, the endoscope main unit 1 is moved so as to follow, in the opposite direction, the same path as the path on which the endoscope main unit 1 was moved while being manually operated by the operator D. Then, as shown in Fig. 12D, in the state in which the operator D has released his/her hand from the operating portion 20, the arm 30 returns the attitude and the position of the endoscope main unit 1 to the initial state at the time when the recording operating mode was started.

Once all arm-angle information that has been recorded in the memory 67 in the recording operating mode has been transmitted to the motors 53 from the motor driver 61 ("YES" in Step SA9), the operating mode is switched to the normal operating mode (Step SA10). When switched to the normal operating mode, the CPU 69 stops supplying VCC from the motor driver 61 to the motors 53 (Step SA11). By doing so, the observation of the desired position in the body cavity by using the endoscope system 100 and the operating method thereof is completed.

As has been described above, with the endoscope system 100 and the operating method thereof according to this embodiment, after the operator D observes the desired position in the body cavity by manually operating the endoscope main unit 1, the endoscope main unit 1 is returned to the original attitude and position, by means of automatic control regardless of the skill level of the operator D, by following the same movement path as when the endoscope main unit 1 was initially moved, and thus, it is possible to obtain a stable viewing field in a highly reproducible manner. Therefore, it is possible to position the endoscope main unit 1 in a highly reproducible manner by reducing cumbersome tasks associated with the operation by the operator D when repeatedly moving the endoscope main unit 1 in a reciprocating manner back and forth between a position close to the affected area and a position overlooking the affected area, between a lateral position for obtaining a viewing field at the side of the affected area and a frontal position, or the like.

### {Second Embodiment}

Next, an endoscope system according to a second embodiment of the present invention will be described.

As shown in Fig. 13, the endoscope system 100 according to this embodiment differs from the first embodiment in that, instead of the control device 50, it is provided with a control device 150 that automatically also restores changes in the shape of the bending portion 11 of the shaft 10 by reproducing the process involved in a reverse order. In the following, the same reference signs are assigned to the portions whose configurations are the same as those of the endoscope system 100 and the operating method thereof according to the first embodiment, and descriptions thereof will be omitted.

Motors 153A, 153B, and 153C that change the rotating angles of each of the distal-end joint portions 13 of the shaft 10 about the respective rotation shafts and encoders 155A, 155B, and 155C that convert the rotating angles of the motors 153A, 153B, and 153C to electrical signals and that output them in the form of arm-angle information are accommodated in an arm-driving apparatus 152.

The control device 150 is additionally provided with a motor driver 161 that drives the motors 153A, 153B, and 153C and the encoders 155A, 155B, and 155C, a VCC supplying portion 163 that outputs VCC (power-source voltage) to be supplied to the motors 153A, 153B, and 153C and the encoders 155A, 155B, and 155C, an A/D converter 165 that converts distal-end-angle information output from the encoders 155A, 155B, and 155C from analog signals and that transmits them to the CPU 69 in the form of digital signals, and a memory (distal-end-angle recording portion) 167 that records the distal-end-angle information (encoder data) for the respective distal-end joint portions 13 transmitted thereto from the CPU 69.

Furthermore, in the normal operating mode, the CPU 69 stops supplying VCC to be sent to the motors 153A, 153B, and 153C from the motor driver 161, while, on the other hand, allowing the VCC to be supplied to the encoders 155A, 155B, and 155C; however, the inputs of the distal-end-angle information from the encoders 155A, 155B, and 155C are stopped.

In addition, in the recording operating mode, the CPU 69 also stops supplying VCC to the motors 153A, 153B, and 153C from the motor driver 161, while, on the other hand, allowing VCC to be supplied also to the encoders 155A, 155B, and 155C from the motor driver 161, receives the inputs of the distal-end-angle information from the encoders 155A, 155B, and 155C, and transmits the information to the memory 167.

In addition, in the automatic operating mode, the CPU 69 allows VCC to be supplied also to the motors 153 and the encoders 155A, 155B, and 155C from the motor driver 161. In addition, in the automatic operating mode, the CPU 69 reads out, in time-series, the distal-end-angle information recorded in the memory 167 in the recording operating mode and transmits the information to the motors 153A, 153B, and 153C from the motor driver 161, and also receives the inputs of the distal-end-angle information from the encoders 155A, 155B, and 155C and transmits the information to the memory 167.

Specifically, when the recording operating mode is switched to the automatic operating mode via the mode-switching switch 23, the CPU 69 makes the motor driver 161 and the motors 153A, 153B, and 153C also reproduce the changes in the rotating angles of each of the distal-end joint portions 13, which have been recorded in the memory 167 in the recording operating mode, in time-series in reverse order. By doing so, the CPU 69 makes the attitude and the position of the endoscope main unit 1, including the changes in the shape of the bending portion 11 of the shaft 10, return to the initial state of the recording operating mode.

With the thus-configured endoscope system 100 according to this embodiment, in the case in which the use of a viewing field located at the back side of tissue or in a narrow space is easily ensured by bending the distal end of the shaft 10 in the direction that intersects the optical axis by using the plurality of distal-end joint portions 13, the endoscope main unit 1 is returned to the original attitude and position, including the bending motion of the shaft 10, regardless of the skill level of the operator D, and thus, it is possible to obtain a stable viewing field in a highly reproducible manner. Therefore, it is possible to position the endoscope main unit 1 in a more reproducible manner by reducing cumbersome tasks associated with the operation by the operator D when repeatedly moving the endoscope main unit 1 in a reciprocating manner.

The individual embodiments described above can be modified as follows. In the individual embodiments described above, although the operating mode is switched among three modes, namely, the normal operating mode, the recording operating mode, and the automatic operating mode, alternatively, as shown in Fig. 14, the first modification may include a manual operating mode (non-recording mode) in which the CPU 69 stops supplying VCC to the motors 53 (153) and the encoders 55 (155) from the motor driver 61 (161). In this case, the mode-switching switch 23 should be used so that the operating mode can be switched among the non-recording mode, the normal operating mode, the recording operating mode, and the automatic operating mode.

By doing so, in the manual operating mode, it is possible to suppress the power required to change the rotating angles of the arm-joint portions 35, 45A, 45B, 45C, and 49 and those of the distal-end joint portions 13 and also to keep the memory capacity of the memories 67 and 167 low. In addition, it is possible for the operator D to manually operate the endoscope main unit 1 without being affected by the CPU 69 by holding the operating portion 20.

### {Third Embodiment}

Next, an endoscope system according to a third embodiment of the present invention will be described.

As shown in Figs. 15A to 15C, an endoscope system 200 according to this embodiment differs from the second embodiment in that, instead of the control device 150, it is provided with a control device 250 that automatically restores the attitude and the position of the endoscope main unit 1 by reproducing, in reverse order, the changes in the rotating angles of the arm-joint portions 35, 45A, 45B, 45C, and 49 and those of the distal-end joint portions 13 based on the image acquired by the image-acquisition unit 15, arm-angle information of the arm-joint portions 35, 45A, 45B, 45C, and 49, and the distal-end-angle information of the distal-end joint portions 13. In the following, the same reference signs are assigned to the portions whose configurations are the same as those of the endoscope system 100 according to the second embodiment, and descriptions thereof will be omitted.

The endoscope system 200 according to this embodiment is provided with an image-acquisition apparatus 210 having the image-acquisition unit 15 and a video-image displaying apparatus 7 that processes the image acquired by the image-acquisition apparatus 210 and that displays it on the monitor 5.

The image-acquisition apparatus 210 is provided with the image-acquisition unit 15, an image-acquisition control portion 211 that controls the image-acquisition unit 15, a signal processing portion 213 that converts an image acquired by the image-acquisition unit 15 to video-image signals, an image processing portion 215 that processes the image, and a video-image-signal outputting portion 217 that transmits the video-image signals obtained by the signal processing portion 213 to the video-image displaying apparatus 7. The image signals obtained by the image processing portion 213 are transmitted to the CPU 69.

The video-image displaying apparatus 7 is provided with the monitor 5, a video-image-signal inputting portion 221 that receives the inputs of the video-image signals transmitted thereto from the image-acquisition apparatus 210, a signal processing portion 223 that processes the video-image signals input to the video-image-signal inputting portion 221, and a monitor control portion 225 that controls the monitor 5.

The control device 250 is provided with an image memory (image recording portion) 251 that records an image of the viewing field at the distal end of the shaft 10 acquired by the image-acquisition unit 15 and an image judging portion (image comparing portion) 253 that compares the image recorded in the image memory 251 in the recording operating mode and an image of the viewing field at the distal end of the shaft 10 acquired by the image-acquisition unit 15 in the automatic operating mode. The image judging portion 253 compares, for example, characteristics of the two images, such as luminance distributions or the like.

The CPU 69 transmits the image signals transmitted thereto from the image processing portion 213 to the image memory 251 via the image judging portion 253. In addition, in the manual operating mode, in the case in which the image judging portion 253 judges that the difference between the characteristic of the image of the viewing filed at the distal end of the shaft 10 acquired in the automatic operating mode and the characteristic of the image recorded by the image memory 251 in the manual operating mode is equal to or greater than a predetermined threshold, the CPU 69 stops reproducing, in reverse order, the rotational driving actions of the arm-joint portions 35, 45A, 45B, 45C, and 49 and those of the distal-end joint portions 13.

The operation of the thus-configured endoscope system 200 will be described based on the flowchart in Fig. 16. In order to observe a desired position in the body cavity by using the endoscope system 200 according to this embodiment, first, the operator D holds and operates the endoscope main unit 1, which is supported by the arm 30, in the normal operating mode (Step SA1), inserts the shaft 10 into the body cavity, and switches the operating mode to the recording operating mode ("YES" in Step SA2).

In the endoscope-holding apparatus 3, by switching to the recording operating mode, via the CPU 69, the arm-angle information of the respective arm-joint portions 35, 45A, 45B, 45C, and 49 output from the encoders 55 in association with the changes in the rotating angles of the individual arm-joint portions 35, 45A, 45B, 45C, and 49 is recorded in the memory 67, and the distal-end-angle information of the respective distal-end joint portions 13 output from the encoders 155 in association with the changes in the rotating angles of the distal-end joint portions 13 is recorded in the memory 167 (Step SA3, recording step).

By doing so, the arm-angle information is recorded in the memory 67 at certain time intervals and the distal-end-angle information is also recorded in the memory 167 at the certain time intervals, using, as the initial points, the arm-angle information of the respective arm-joint portions 35, 45A, 45B, 45C, and 49 and the distal-end-angle information of the respective distal-end joint portions 13 at the time when the recording operating mode is started.

In addition, the image signals that are acquired by the image-acquisition unit 15 and that are transmitted from the image processing portion 213 are recorded in the image memory 251 via the CPU 69 (Step SB3) in synchronization with respective recording cycles of the arm-angle information and the distal-end-angle information recorded in the memory 67 and the memory 167.

Subsequently, when the image acquisition at a desired observation position in the body cavity is completed and when the operator D switches the operating mode to the automatic operating mode by releasing his/her finger from the mode-switching switch 23 (Step SA4), the CPU 69 allows VCC to be supplied to the motors 53 and 153 and the encoders 55 and 155 from the motor drivers 61 and 161 (Step SA5). Then, the CPU 69 reads out, in time-series, the image signals recorded in the manual operating mode from the image memory 251 (Step SB6) .

In addition, the CPU 69 reads out, in time-series, the arm-angle information and the distal-end-angle information recorded in the memory 67 and the memory 167 in recording operating mode (Step SA6). Then, the image judging portion 253 compares the image signals acquired in the automatic operating mode and the image signals recorded in the memory in the recording operating mode, and judges whether or not the characteristics of these image signals match with each other (Step SB7).

In the case in which the image judging portion 253 judges that the two sets of image data match with each other, the CPU 69 causes the read-out arm-angle information to be transmitted, in time-series, to the arm-joint portions 35, 45A, 45B, 45C, and 49 and the distal-end joint portions 13 from the motor drivers 61 and 161 (Step SA7), the individual motors 53 are driven based on the arm-angle information, and, in addition, the individual motors 153 are driven based on the distal-end-angle information (Step SA8, automatic restoring step).

By doing so, the changes in the rotating angles of each of the arm-joint portions 35, 45A, 45B, 45C, and 49 and the distal-end joint portions 13 in the recording operating mode are reproduced in time-series in the reverse order. Thus, the endoscope main unit 1 is moved so as to follow, in the opposite direction, the same path as the path on which the endoscope main unit 1 was moved while being manually operated by the operator D, and the arm 30 returns the attitude and the position of the endoscope main unit 1 to the initial state at the time when the recording operating mode was started.

Once all arm-angle information and distal-end-angle information recorded in the memory 67 and the memory 167 in the recording operating mode have been transmitted to the motors 53 and 153 from the motor drivers 61 and 161 ("YES" in Step SA9), the mode-switching switch 23 is switched to the normal operating mode (Step SA10). On the other hand, if all arm-angle information has not been transmitted to the motors 53 and 153 from the motor drivers 61 and 161, the procedure returns to Step SB6.

When switched to the normal operating mode, the CPU 69 stops supplying VCC to the motors 53 and 153 from the motor drivers 61 and 161 (Step SA11). By doing so, the observation of the desired position in the body cavity by using the endoscope system 200 and the operating method thereof is completed.

On the other hand, in Step SB7, in the case in which the image judging portion 253 judges that the difference between the two sets of the image data is equal to or greater than the predetermined threshold, the CPU 69 switches the operating mode to the normal operating mode (Step SA10) and stops supplying VCC to the motors 53 and 153 from the motor drivers 61 and 161. By doing so, reproduction of the rotational driving actions of the arm-joint portions 35, 45A, 45B, 45C, and 49 and those of the distal-end joint portions 13 in the reverse order is stopped.

As has been described above, with the endoscope system 200 according to this embodiment, in the case in which the image of the viewing field at the distal end of the shaft 10 acquired in the automatic operating mode does not match with the image recorded in the recording operating mode, because the CPU 69 stops reproducing, in the reverse order, the rotational driving actions of the arm-joint portions 35, 45A, 45B, 45C, and 49 and those of the distal-end joint portions 13, it is possible to prevent the shaft 10 from coming into contact with a treatment tool or the like even if there have been changes in the environment in the body cavity, states of other treatment tools, or the like between the recording operating mode and the automatic operating mode.

The individual embodiments described above can be modified as follows. For example, as shown in Fig. 17, a plurality of endoscope main units 1 may have ID recording portions 219 that record unit-specific information in accordance with the types, numbers, and positions of the arm-joint portions 35, 45A, 45B, 45C, and 49 and the distal-end joint portions 13.

In addition, as shown in Fig. 18, the arm 30 may be provided with an ID reading portion (identifying portion) 239 that identifies the unit-specific information of the endoscope main unit 1 supported by the arm 30. Also, the CPU 69 may control changes in the rotating angles of each of the arm-joint portions 35, 45A, 45B, 45C, and 49 and the distal-end joint portions 13 based on the unit-specific information of the ID recording portion 219 identified by the ID reading portion 239.

In this case, the ID recording portion 219 should be disposed at an outer circumferential surface of the shaft 10 at a position that is held by the holding portion 31.

In addition, the ID reading portion 239 should employ, for example, a non-contact RFID (Radio Frequency Identification) device that is disposed at a position, facing the ID recording portion 219 of the held shaft 10, in the through-hole 31a of the holding portion 31.

By doing so, when the shaft 10 of the endoscope main unit 1 is inserted into the through-hole 31a of the holding portion 31 of the arm 30, the ID reading portion 239 identifies the unit-specific information in the ID recording portions 219 for the respective endoscope main units 1, and the CPU 69 controls the changes in the rotating angles of each of the arm-joint portions 35, 45A, 45B, 45C, and 49 and the distal-end joint portions 13 based on that unit-specific information. Therefore, via the CPU 69, it is possible to perform operations in accordance with the types, numbers, and positions of the arm-joint portions 35, 45A, 45B, 45C, and 49 and the distal-end joint portions 13 for each endoscope main unit 1.

In this modification, although the unit-specific information in accordance with the types, numbers, and positions of the arm-joint portions 35, 45A, 45B, 45C, and 49 and the distal-end joint portions 13 has been described as an example, the unit-specific information is not limited thereto.

Although the embodiments of the present invention have been described above in detail with reference to the drawings, specific configurations are not limited to these embodiments, and design alterations or the like that do not depart from the scope of the present invention are also encompassed. For example, the present invention is not limited to inventions applied to the individual embodiments and modifications described above, and the present invention may be applied to an embodiment in which these embodiments and modifications are appropriately combined, without particular limitation.

In the individual embodiments described above, although the arm 30 has been described as an example of a holder, it suffices that a holder supports the endoscope main unit 1, has a plurality of holder joint portions whose rotating angles about respective predetermined rotation shafts can be changed, and changes the attitude and the position of the endoscope main unit 1 by changing the rotating angles of the holder joint portions, and the holder need not have an arm shape. In addition, although the arm-joint portions 35, 45A, 45B, 45C, and 49 have been described as examples of the holder joint portions, the type and the number of the holder joint portions are not limited thereto.

### {Reference Signs List}

1 endoscope main unit
10 shaft (inserted portion)
13 distal-end joint portion
15 image-acquisition unit
20 operating portion (main-unit operating portion)
21 joystick (distal-end operating portion)
23 mode-switching switch (switching portion)
35 endoscope-rotating joint portion (holder joint portion)
30 arm (holder)
45A, 45B, 45C bending joint portion (holder joint portion)
49 arm-rotating joint portion (holder-rotating joint portion)
53, 153 motor (rotational drive portion)
57, 157 encoder (information outputting portion)
67 memory (holder-angle recording portion)
69 CPU (control portion)
100, 200 endoscope system
167 memory (distal-end-angle recording portion)
251 image memory (image recording portion)
253 image judging portion (image comparing portion)
239 ID reading portion (identifying portion)
SA3 recording step
SA8 automatic restoring step

## Claims

1. An endoscope system (100) comprising:
an endoscope main unit (1) provided with an insertion portion (10) that is adapted to be inserted into a body cavity, a main-unit operating portion (20) that is provided at a proximal end of the insertion portion and that can be held by an operator, and an image-acquisition unit (15) that acquires an image of a viewing field at a distal end of the insertion portion;
a holder (30) that supports the endoscope main unit (1), that has a plurality of holder joint portions (35, 45A, 45B, 45C) whose rotating angles can be changed about respective predetermined rotation shafts, and that can change an attitude and a position of the endoscope main unit (1) by changing the rotating angles of the holder joint portions;
a holder-angle recording portion (67) that records holder-angle information regarding the rotating angles of the individual holder joint portions;
a control portion (69) that controls changes in the rotating angles of the individual holder joint portions; and
a switching portion (23) that switches between a manual mode in which the operator holds the main-unit operating portion and changes the attitude and the position of the endoscope main unit and an automatic restoring mode in which the attitude and the position of the endoscope main unit are changed by the control portion,
wherein, in the case in which the operating mode is switched to the automatic restoring mode from the manual mode by using the switching portion, the control portion reproduces changes in the rotating angles of the individual holder joint portions in time-series in a reverse order based on the holder-angle information recorded in the holder-angle recording portion in the manual mode, thus returning the attitude and the position of the endoscope main unit to an initial state of the manual mode,
**characterized in that** the endoscope main unit includes:
a plurality of distal-end joint portions (13) that are provided at a distal-end portion of the insertion portion and whose rotating angles can be changed about respective predetermined rotation shafts that intersect the longitudinal direction; and
a distal-end operating portion (150) that changes the rotating angles of the respective distal-end joint portions (13) in response to an operation by the operator,
wherein the endoscope system (100) comprises a distal-end-angle recording portion (167) that records distal-end-angle information regarding time-series changes in the rotating angles of the respective distal-end joint portions (13) that are changed by the distal-end operating portion, and
wherein, in the case in which the operating mode is switched to the automatic restoring mode from the manual mode by using the switching portion (23), the control portion reproduces changes in the rotating angles of the respective distal-end joint portions in time-series in a reverse order, in synchronization with reproducing the changes in the rotating angles of the individual holder joint portions in a reverse order, based on the distal-end-angle information recorded in the distal-end-angle recording portion (167) in the manual mode, thus returning the angle of the insertion portion to the initial state of the manual mode.

2. An endoscope system according to Claim 1, wherein the plurality of holder joint portions include:
a plurality of bending joint portions (45A, 45B, 45C) that can be rotated about the rotation shafts that intersect a longitudinal direction of the holder;
a holder-rotating joint portion that can be rotated about the rotation shaft that extends in the longitudinal direction of the holder; and
an endoscope-rotating joint portion (35) that rotates the insertion portion about the rotation shaft that extends along an optical axis of the image-acquisition unit.

3. An endoscope system according to Claim 1 or 2, wherein the holder joint portions include:
rotational drive portions that are rotationally driven about the rotation shafts; and
an information outputting portion that outputs the holder-angle information of the rotational drive portions,
wherein the control portion stops control of the rotational drive portions in the manual mode, and stops outputting of the holder-angle information from the information outputting portion in the automatic restoring mode.

4. An endoscope system according to any one of Claims 1 to 3, wherein the switching portion stops the control of the individual holder joint portions by the control portion, also stops recording of the holder-angle information by the holder-angle recording portion, and switches the operating mode among a non-recording mode in which the operator holds the main-unit operating portion and changes the attitude and the position of the endoscope main unit, the manual mode, and the automatic restoring mode.

5. An endoscope system according to any one of Claims 1 to 4, further comprising:
an image recording portion (251) that records an image of a viewing field at the distal end, acquired by the image-acquisition unit; and
an image comparing portion (253) that compares the image recorded by the image recording portion in the manual mode and an image of the viewing field at the distal end, acquired by the image-acquisition unit in the automatic restoring mode,
wherein, in the case in which the image comparing portion judges that a difference between a characteristic of the image of the viewing field at the distal end, acquired in the automatic restoring mode, and a characteristic of the image recorded by the image recording portion in the manual mode is equal to or greater than a predetermined threshold, the control portion stops reproducing the changes in the rotating angles of the holder joint portions in the reverse order.

6. An endoscope system according to any one of Claims 1 to 5,
wherein a plurality of the endoscope main units in which types, numbers, and/or positions of the holder joint portions are different have unit-specific information in accordance with the types, numbers, and/or positions of the holder joint portions,
the holder is provided with an identifying portion that identifies the unit-specific information of the supported endoscope apparatus, and
the control portion controls changes in the rotating angles of the individual holder joint portions based on the unit-specific information identified by the identifying portion.

## Patentansprüche

1. Endoskopsystem (100), umfassend:
eine Endoskophaupteinheit (1), die mit einem Einführabschnitt (10), der dazu angepasst ist, in eine Körperhöhle eingesetzt zu werden, einem Haupteinheitbetriebsabschnitt (20), der an einem proximalen Ende des Einführabschnitts vorgesehen ist und der von einem Bediener gehalten werden kann, und einer Bildaufnahmeeinheit (15), die ein Bild eines Sichtfeldes an einem distalen Ende des Einführabschnitts aufnimmt, versehen ist;
eine Halterung (30), die die Endoskophaupteinheit (1) stützt, die eine Mehrzahl von Halterungsverbindungsabschnitten (35, 45A, 45B, 45C) aufweist, deren Drehwinkel um entsprechende vorbestimmte Drehwellen geändert werden können, und die eine Lage und eine Position der Endoskophaupteinheit (1) durch Ändern der Drehwinkel der Halterungsverbindungsabschnitte ändern kann;
einen Halterungswinkelaufzeichnungsabschnitt (67), der eine Halterungswinkelinformation mit Bezug auf die Drehwinkel der einzelnen Halterungsverbindungsabschnitte aufzeichnet;
einen Steuerungsabschnitt (69), der Änderungen in den Drehwinkeln der einzelnen Halterungsverbindungsabschnitte ändert; und
einen Schaltabschnitt (23), der zwischen einem manuellen Modus, in dem der Bediener den Haupteinheitsbetriebsabschnitt hält und die Lage und die Position der Endoskophaupteinheit ändert, und einem automatischen Wiederherstellungsmodus, in dem die Lage und die Position der Endoskophaupteinheit durch den Steuerungsabschnitt geändert werden, schaltet,
wobei, in dem Fall, in dem der Betriebsmodus mithilfe des Schaltabschnitts von dem manuellen Modus auf den automatischen Wiederherstellungsmodus geschaltet wird, der Steuerungsabschnitt Änderungen in den Drehwinkeln der einzelnen Halterungsverbindungsabschnitte in Zeitreihen in einer umgekehrten Reihenfolge basierend auf der Halterungswinkelinformation, die in dem Halterungswinkelaufzeichnungsabschnitt in dem manuellen Modus aufgezeichnet wurden, reproduziert, wodurch die Lage und die Position der Endoskophaupteinheit auf einen Anfangszustand des manuellen Modus zurückkehren,
**dadurch gekennzeichnet, dass** die Endoskophaupteinheit umfasst:
eine Mehrzahl von Distalendverbindungsabschnitten (13), die an einem Distalendabschnitt des Einführabschnitts vorgesehen sind und deren Drehwinkel um entsprechende vorbestimmte Drehwellen geändert werden können, die die Längsrichtung schneiden; und
einen Distalendbetriebsabschnitt (150), der die Drehwinkel der entsprechenden Distalendverbindungsabschnitte (13) in Reaktion auf einen Betrieb durch den Bediener ändert,
wobei das Endoskopsystem (100) einen Distalendwinkelaufzeichnungsabschnitt (167) umfasst, der eine Distalendwinkelinformation in Bezug auf Zeitreihenänderungen in den Drehwinkeln der entsprechenden Distalendverbindungsabschnitte (13) aufzeichnet, die durch den Distalendbetriebsabschnitt geändert werden, und
wobei, in dem Fall, in dem der Betriebsmodus mithilfe des Schaltabschnitts (23) von dem manuellen Modus auf den automatischen Wiederherstellungsmodus geschaltet wird, der Steuerungsabschnitt Änderungen in den Drehwinkeln der entsprechenden Distalendverbindungsabschnitte in Zeitreihen in einer umgekehrten Reihenfolge synchron zum Reproduzieren der Änderungen in den Drehwinkeln der einzelnen Halterungsverbindungsabschnitte in einer umgekehrten Reihenfolge basierend auf der in dem Distalendwinkelaufzeichnungsabschnitt (167) aufgezeichneten Distalendwinkelinformation reproduziert, wodurch der Winkel des Einführabschnitts auf den Anfangszustand des manuellen Modus zurückkehrt.

2. Endoskopsystem nach Anspruch 1, wobei die Mehrzahl von Halterungsverbindungsabschnitten umfasst:
eine Mehrzahl von Biegeverbindungsabschnitten (45A, 45B, 45C), die um die Drehwellen gedreht werden können, die eine Längsrichtung der Halterung schneiden;
einen Halterungsdrehverbindungsabschnitt, der um die Drehwelle gedreht werden kann, die sich in die Längsrichtung der Halterung erstreckt; und
einen Endoskopdrehverbindungsabschnitt (35), der den Einführabschnitt um die Drehwelle dreht, die sich entlang einer optischen Achse der Bildaufnahmeeinheit erstreckt.

3. Endoskopsystem nach Anspruch 1 oder 2, wobei die Halterungsverbindungsabschnitte umfassen:
Drehantriebsabschnitte, die um die Drehwellen drehangetrieben werden; und
einen Informationsausgabeabschnitt, der die Halterungswinkelinformation der Drehantriebsabschnitte ausgibt,
wobei der Steuerungsabschnitt die Steuerung der Drehantriebsabschnitte in dem manuellen Modus beendet und das Ausgeben der Halterungswinkelinformation aus dem Informationsausgabeabschnitt in dem automatischen Wiederherstellungsmodus beendet.

4. Endoskopsystem nach einem der Ansprüche 1 bis 3, wobei der Schaltabschnitt die Steuerung der einzelnen Halterungsverbindungsabschnitte durch den Steuerungsabschnitt beendet, ebenfalls das Aufzeichnen der Halterungswinkelinformation durch den Halterungswinkelaufzeichnungsabschnitt beendet und den Betriebsmodus zwischen einem Nichtaufzeichnungsmodus, in dem der Bediener den Haupteinheitbetriebsabschnitt hält und die Lage und die Position der Endoskophaupteinheit ändert, dem manuellen Modus und dem automatischen Wiederherstellungsmodus schaltet.

5. Endoskopsystem nach einem der Ansprüche 1 bis 4, ferner umfassend:
einen Bildaufzeichnungsabschnitt (251), der ein Bild eines Sichtfeldes an dem distalen Ende aufzeichnet, das von der Bildaufnahmeeinheit aufgenommen wird; und
einen Bildvergleichsabschnitt (253), der das von dem Bildaufzeichnungsabschnitt in dem manuellen Modus aufgezeichnete Bild und ein Bild des Sichtfeldes an dem distalen Ende, das von der Bildaufnahmeeinheit in dem automatischen Wiederherstellungsmodus aufgenommen wurde, vergleicht,
wobei in dem Fall, in dem der Bildvergleichsabschnitt bewertet, dass ein Unterschied zwischen einer Eigenschaft des Bildes des Sichtfeldes an dem distalen Ende, das in dem automatischen Wiederherstellungsmodus aufgenommen wurde, und einer Eigenschaft des von dem Bildaufzeichnungsabschnitt in dem manuellen Modus aufgezeichneten Bildes gleich oder größer ist als eine vorbestimmte Schwelle, der Steuerungsabschnitt das Reproduzieren der Änderungen in den Drehwinkeln der Halterungsverbindungsabschnitte in der umgekehrten Reihenfolge beendet.

6. Endoskopsystem nach einem der Ansprüche 1 bis 5,
wobei eine Mehrzahl der Endoskophaupteinheiten, bei denen sich Typ, Anzahl und/oder Position der Halterungsverbindungsabschnitte unterscheiden, eine einheitenspezifische Information entsprechend des Typs, der Anzahl und/oder Positionen der Halterungsverbindungsabschnitte aufweist,
die Halterung mit einem Identifizierungsabschnitt versehen ist, der die einheitenspezifische Information der unterstützten Endoskopvorrichtung identifiziert, und
der Steuerungsabschnitt Änderungen in den Drehwinkeln der einzelnen Halterungsverbindungsabschnitte basierend auf der von dem Identifizierungsabschnitt identifizierten einheitenspezifischen Information steuert.

## Revendications

1. Système d'endoscope (100) comprenant :
une unité principale d'endoscope (1) comportant une partie d'insertion (10) qui est apte à être insérée dans une cavité corporelle, une partie d'actionnement d'unité principale (20) qui est prévue à une extrémité proximale de la partie d'insertion et qui peut être maintenue par un opérateur, et une unité d'acquisition d'image (15) qui acquiert une image d'un champ de vision à une extrémité distale de la partie d'insertion ;
un support (30) qui supporte l'unité principale d'endoscope (1), qui a une pluralité de parties d'articulation de support (35, 45A, 45B, 45C) dont des angles de rotation peuvent être changés autour de tiges de rotation prédéterminées respectives, et qui peut changer une attitude et une position de l'unité principale d'endoscope (1) par changement des angles de rotation des parties d'articulation de support ;
une partie d'enregistrement d'angle de support (67) qui enregistre des informations d'angle de support concernant les angles de rotation des parties d'articulation de support individuelles ;
une partie de commande (69) qui commande des changements des angles de rotation des parties d'articulation de support individuelles ; et
une partie de commutation (23) qui commute entre un mode manuel, dans lequel l'opérateur maintient la partie d'actionnement d'unité principale et change l'attitude et la position de l'unité principale d'endoscope, et un mode de rappel automatique, dans lequel l'attitude et la position de l'unité principale d'endoscope sont changées par la partie de commande,
dans le cas où le mode d'actionnement est commuté au mode de rappel automatique à partir du mode manuel par utilisation de la partie de commutation, la partie de commande reproduisant des changements des angles de rotation des parties d'articulation de support individuelles en série chronologique dans un ordre inverse sur la base des informations d'angle de support enregistrées dans la partie d'enregistrement d'angle de support dans le mode manuel, ramenant ainsi l'attitude et la position de l'unité principale d'endoscope à un état initial du mode manuel,
**caractérisé par le fait que** l'unité principale d'endoscope comprend :
une pluralité de parties d'articulation d'extrémité distale (13) qui sont prévues à une partie extrémité distale de la partie d'insertion et dont les angles de rotation peuvent être changés autour de tiges de rotation prédéterminées respectives qui coupent la direction longitudinale ; et
une partie d'actionnement d'extrémité distale (150) qui change les angles de rotation des parties d'articulation d'extrémité distale respectives (13) en réponse à un actionnement par l'opérateur,
le système d'endoscope (100) comprenant une partie d'enregistrement d'angle d'extrémité distale (167) qui enregistre des informations d'angle d'extrémité distale concernant des changements, en série chronologique, des angles de rotation des parties d'articulation d'extrémité distale respectives (13) qui sont changés par la partie d'actionnement d'extrémité distale, et
dans le cas où le mode d'actionnement est commuté au mode de rappel automatique à partir du mode manuel par utilisation de la partie de commutation (23), la partie de commande reproduisant des changements des angles de rotation des parties d'articulation d'extrémité distale respectives en série chronologique dans un ordre inverse, en synchronisation avec la reproduction des changements des angles de rotation des parties d'articulation de support individuelles dans un ordre inverse, sur la base des informations d'angle d'extrémité distale enregistrées dans la partie d'enregistrement d'angle d'extrémité distale (167) dans le mode manuel, ramenant ainsi l'angle de la partie d'insertion à l'état initial du mode manuel.

2. Système d'endoscope selon la revendication 1, dans lequel la pluralité de parties d'articulation de support comprennent :
une pluralité de parties d'articulation de courbure (45A, 45B, 45C) qui peuvent être tournées autour des tiges de rotation qui coupent une direction longitudinale du support ;
une partie d'articulation de rotation de support qui peut être tournée autour de la tige de rotation qui s'étend dans la direction longitudinale du support ; et
une partie d'articulation de rotation d'endoscope (35) qui tourne la partie d'insertion autour de la tige de rotation qui s'étend le long d'un axe optique de l'unité d'acquisition d'image.

3. Système d'endoscope selon la revendication 1 ou 2, dans lequel les parties d'articulation de support comprennent :
des parties d'entraînement en rotation qui sont entraînées en rotation autour des tiges de rotation ; et
une partie de sortie d'informations qui délivre en sortie les informations d'angle de support des parties d'entraînement en rotation,
la partie de commande arrêtant une commande des parties d'entraînement en rotation dans le mode manuel et arrêtant la sortie des informations d'angle de support par la partie de sortie d'informations dans le mode de rappel automatique.

4. Système d'endoscope selon l'une quelconque des revendications 1 à 3, dans lequel la partie de commutation arrête la commande des parties d'articulation de support individuelles par la partie de commande, arrête également l'enregistrement des informations d'angle de support par la partie d'enregistrement d'angle de support, et commute le mode d'actionnement parmi un mode de non-enregistrement dans lequel l'opérateur maintient la partie d'actionnement d'unité principale et change l'attitude et la position de l'unité principale d'endoscope, le mode manuel et le mode de rappel automatique.

5. Système d'endoscope selon l'une quelconque des revendications 1 à 4, comprenant en outre :
une partie d'enregistrement d'image (251) qui enregistre une image d'un champ de vision à l'extrémité distale, acquise par l'unité d'acquisition d'image ; et
une unité de comparaison d'images (253) qui compare l'image enregistrée par la partie d'enregistrement d'image dans le mode manuel et une image du champ de vision à l'extrémité distale, acquise par l'unité d'acquisition d'image dans le mode de rappel automatique,
dans le cas où la partie de comparaison d'images détermine qu'une différence entre une caractéristique de l'image du champ de vision à l'extrémité distale, acquise dans le mode de rappel automatique, et une caractéristique de l'image enregistrée par la partie d'enregistrement d'image dans le mode manuel est supérieure ou égale à un seuil prédéterminé, la partie de commande arrêtant la reproduction des changements des angles de rotation des parties d'articulation de support dans l'ordre inverse.

6. Système d'endoscope selon l'une quelconque des revendications 1 à 5,
dans lequel une pluralité d'unités principales d'endoscope, dans lesquelles des types, nombres et/ou positions des parties d'articulation de support sont différents, ont des informations spécifiques à une unité en fonction des types, nombres et/ou positions des parties d'articulation de support,
le support comporte une partie d'identification qui identifie les informations spécifiques à l'unité de l'appareil d'endoscope supporté, et
la partie de commande commande des changements des angles de rotation des parties d'articulation de support individuelles sur la base des informations spécifiques à l'unité identifiées par la partie d'identification.
